# EUROPEAN PATENT APPLICATION

(11) **EP 2 417 987 A2**
(43) Date of publication of application: **15.02.2012**
(21) Application number: 11250726.4
(22) Date of filing: 12.08.2011
(51) Int. Cl.: A61L 17/12

(54) **Surface eroding sutures**

(30) Priority: 13.08.2010 US 856202
(71) Applicant: Tyco Healthcare Group LP, New Haven, CT 06511 (US)
(72) Inventor: Stopek, Joshua, Guilford, CT 06437 (US)
(74) Representative: Soames, Candida Jane

(57) **Abstract**

Barbed surgical sutures comprised of surface eroding materials are provided having increased degradation rates are provided.

## Description

### CROSS-REFERENCE TO RELATED APPLICATION

This application is a continuation-in-part of U.S. Patent Application No. 11/673,073 filed on February 9, 2007, the entire contents of which are incorporated by reference herein.

### TECHNICAL FIELD

The present disclosure relates to surface eroding sutures having an adjustable surface erosion rate.

### BACKGROUND OF RELATED ART

Barbed sutures, which are generally made of the same materials as conventional sutures, offer several advantages for closing wounds compared with conventional sutures. A barbed suture includes an elongate body that has one or more spaced barbs, which project from the surface of the suture body along the body length. The barbs are arranged to allow passage of the barbed suture in one direction through tissue but resist movement of the barbed suture in the opposite direction. Thus, one advantage of barbed sutures has been the decreased time in closing wounds due to less knot tying by the surgeon.

Barbed sutures are known for use in cosmetic, laparoscopic and endoscopic procedures. Using barbed sutures enables the placement of tension in tissue with less slippage of the suture in the wound. Similar to a conventional suture, a barbed suture may be inserted into tissue using a surgical needle.

Depending on the tissue and wound type, patient health and corresponding healing rates, there is an optimal time needed for sutures to be present in the patient. Current sutures provide strength for a given period of time, however, suture mass is still present weeks after body no longer requires them to be. For example, a suture may provide strength for 10 days but is not completely absorbed by the body until 56 days. This foreign body may interfere with healing as it continues to degrade well after suture strength is needed. Therefore, it may be advantageous to incorporate surface eroding polymers in barbed sutures whose properties can provide necessary strength and appropriate mass loss, enabling the wound healing process.

### SUMMARY

A process of the present disclosure may include contacting at least one hydrophobic core with at least one α-hydroxy acid ester to form at least one macromer, contacting the at least one macromer with at least one diacid spacer to form a surface eroding material and forming a suture from the surface eroding material, the suture including an elongate body with a proximal end and a distal end.

In embodiments, a process of the present disclosure may includes contacting at least one hydrophobic core with at least one α-hydroxy acid ester to form at least one macromer, contacting the at least one macromer with at least one diacid spacer to form a surface eroding material, forming a suture from the surface eroding material, the suture having an elongate body with a proximal end and a distal end; and forming a plurality of surface features on the elongate body.

### BRIEF DESCRIPTION OF THE DRAWINGS

Various embodiments of the present disclosure will be described hereinbelow with reference to the figures wherein:

Fig. 1 is a schematic diagram of a surface eroding material in accordance with the present disclosure;

Fig. 2 is a flow diagram of synthesis of a surface eroding polymer in accordance with the present disclosure;

Fig. 3 is a perspective view of a unidirectional surface eroding suture in accordance with the present disclosure; and

Fig. 4 is a perspective view of a bidirectional barbed suture in accordance with the present disclosure.

### DETAILED DESCRIPTION

Described herein is a surface eroding surgical suture including a body and a plurality of surface features extending therefrom, such as barbs, hooks, latches, protrusions, leaves, teeth and/or combinations thereof. The suture may be a unidirectional suture, configured to penetrate tissue via its proximal/needled end or a bidirectional suture configured to penetrate tissue via either end of the elongate body.

The suture is formed from a surface eroding material. As used herein, the term "surface eroding" refers to a degradation process of the suture and/or the material from which the suture is made, the degradation occurring from the surface of the suture rather from a bulk erosion of the entire body of the suture, the latter of which results in disproportionate mass loss, such that mass is left behind long after strength loss.

The materials utilized to form the suture are selected to provide the suture with a desired surface eroding profile, mass loss profile, strength retention, and tensile properties. The suture of the present disclosure has an adjustable surface erosion rate which is controlled by modifying the surface area of the suture body, namely, by adjusting the number, structure, shape, and angle of the surface features disposed on the surface thereof

The surface features may project from the elongate body towards one of the proximal or distal ends at an included angle of less than about 90° between the surface features and the suture body to allow movement of the suture through the tissue in one direction while preventing and/or restricting movement in the reverse direction.

A specific rate of degradation of a suture may be based on the time needed for healing a wound during which the rate of mass loss is proportional to the rate of strength loss, such that the suture strength is maintained until the suture is absorbed within a desired time frame. In embodiments, the mass loss and strength loss may occur within about 30 days of each other. Utilizing surface eroding polymers and methods of the present disclosure, the mass loss profile of the suture may be optimized so that the suture is completely degraded at the most appropriate time, which may be of importance in certain major surgeries such as cosmetic, laparoscopic and endoscopic procedures.

The surface eroding suture of the present disclosure may be made of any suitable surface eroding polymers, which may include polyanhydrides and anhydride copolymers having a hydrophobic component, such as 1,6-bis(p-carboxyphenoxy)hexane (CPH) and a hydrophilic component such as 1,8-bis-(p-carboxyphenoxy)-3,6-dioxaoctane (CPTEG) or other oligomer polyethyleneglycol (PEG) containing units.

In embodiments, a suture of the present disclosure may be made of polysalicylic acid, polydiflunisal, polyhyrdoxybutyrates and polyhydroxy alkoanates, polyorthoesters, poly(alpha-hydroxy esters), valerolactone, N-trimethyl chitosan chloride, and dioxanone. Degradable lactones may incorporate the use of silicone oils/oligomers (hydroxyl terminated) as chemical initiator, or D5 cyclic monomer for copolymerization with lactone monomers. In addition, another embodiment includes using an anionic ring opening polymerization of cyclic lactone monomers with oligomeric/cyclic bisphenol A to prepare degradable phenylated carbonates (i.e., TMC with phenol).

In embodiments, the surface eroding suture may be formed from polymers derived from α-hydroxy acids (PHA) such as lactic and glycolic acids. In particular, surface erosion may be achieved in PHA by tuning the hydrophobicity of the polymer backbone by linking together building blocks of varying lipophilicities. In embodiments, macromer diols with differing hydrophobic-lipophilic properties may be chemically coupled with diacid spacers to produce suitable PHAs that exhibit thermal and physical properties, including surface erosion behavior, distinct from pure poly(L-lactic acid) (PLLA) as described in Xu et al., "Towards Developing Surface Eroding Poly(α-hydroxy acids)," Biomaterials 27 (2006), 3021-3030, the entire contents of which are incorporated by reference herein.

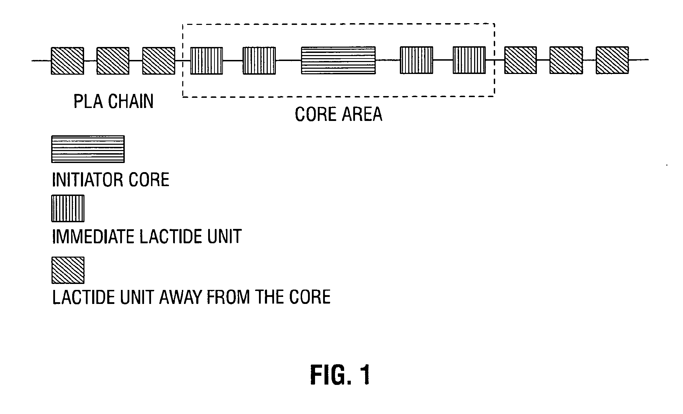

A surface eroding polymer suitable for forming sutures of the present disclosure includes a plurality of macromer diols interconnected with hydrophobic spacers. The macromer is shown in Fig. 1 and includes a core area and a plurality of chains stemming therefrom. The core area includes a hydrophobic initiator core and a plurality of hydrophilic units branching therefrom. The initiator core may be any alkane diol with α and ω-hydroxyl end groups such as 1,2-ethanediol, 1,3-propanediol, 1,4-butanediol, 1,5-pentanediol, 1,6-hexanediol, 1,7-heptanediol, 1,8-octanediol, 1,9-nonanediol, 1,10-decanediol, 1,12-dodecanediol, and the like.

The hydrophilic unit may be any suitable cyclic α-hydroxy acid esters such as lactic acid, lactide mixtures of L-, D-, and meso lactides, glycolic acid, glycolide, epsilon-caprolactone, p-dioxanone (1,4-dioxan-2-one), trimethylene carbonate, delta-valerolactone, beta-butyrolactone, epsilon-decalactone, 2,5-diketomorpholine, pivalolactone, alpha-diethylpropiolactone, ethylene carbonate, ethylene oxalate, 3-methyl-1,4-dioxane-2,5-dione, 3,3-diethyl-1,4-dioxan-2,5-dione, gamma-butyrolactone, 1,4-dioxepan-2-one, 1,5-dioxepan-2-one, 1,4-dioxan-2-one, 6,8-dioxabicycloctane-7-one, and combinations thereof.

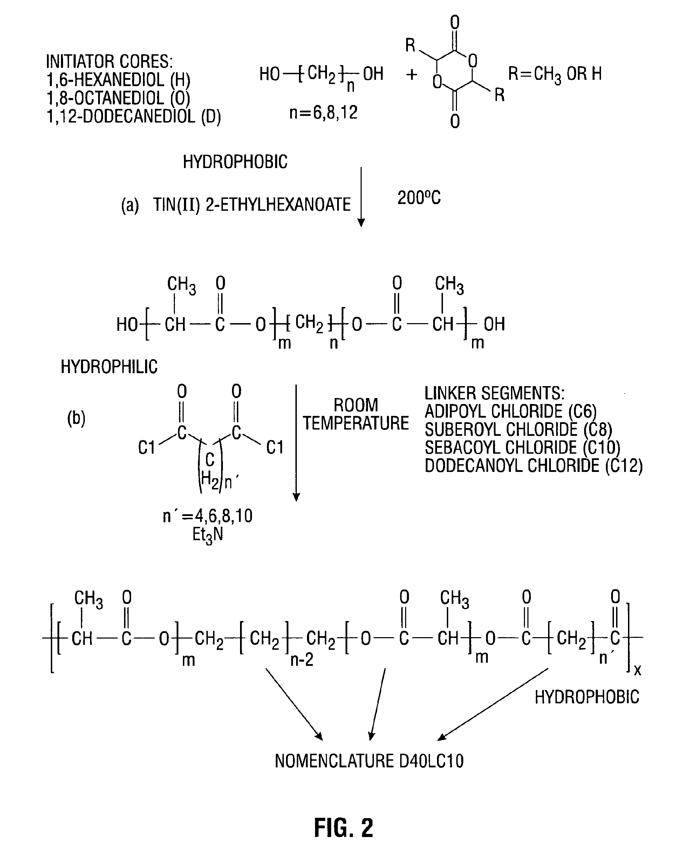

In embodiments, the initiator core is reacted with the cyclic α-hydroxyl acid monomer to obtain macromer diols having hydroxyl end groups. With reference to Fig. 2, the reaction of the alkane diol and the cyclic α-hydroxyl acid ester is a ring opening reaction in the presence of a transesterification catalyst, such as Tin(II) 2-ethylhexanoate. The use of alkane diol initiators results in the formation of symmetrical macromer diols having alkane cores and terminal hydroxyl groups, wherein the degree of polymerization depends on the molar ratio of the cyclic α-hydroxyl acid ester to alkane diol. Varying the carbon chain length of the initiator core and its ratio with the cyclic α-hydroxyl acid ester, macromer diols of varying lipophilic characteristics may be produced.

The surface eroding polyesters are obtained using a condensation polymerization reaction by linking at least tow of the above macromers with a variety of hydrophobic diacid spacers such as diacid dichlorides in the presence of triethylamine. Suitable diacid chlorides include adipoyl chlode, suberoyl chloride, dodecanediol dichloride, and the like. The spacers, sometimes refered to as a hydrophobic spacer is free to react with at least two hydrophilic branches from the at least two different macromers. The resulting polyester includes a hydrophobic core formed of an alkane diol, which is surrounded by hydrophilic units formed of cyclic α-hydroxyl acid esters interspersed with hydrophobic spacers formed of diacid dichlorides. Examples of which surface eroding polyesters include polylactide, polyglycolide, trimethylene carbonate, polycaprolactone, polydioxanone and the like.

The polyester polymers having a general structure shown in Fig. 1 display surface eroding characteristics unlike traditional biocompatible polyesters, such as poly(lactic-co-glycolic acid) (PLGA) and poly(L-lactic acid) (PLLA), which makes them suitable for forming surface eroding sutures. Without being constrained by any particular theory, it is believed that the unique surface eroding characteristics of the polyester polymers according to the present disclosure are due to the combination of hydrophobic and hydrophilic units within the polyester. In particular, it is believed that diminished crystallinity of corresponding macromer diols are advantageous from an erosion standpoint, as crystalline domains degrade slower in comparison with amorphous resin of traditional poly(lactic-co-glycolic acid) and poly(L-lactic acid). Due to fairly hydrophobic nature of the polyester as compared with PLGA and PLLA, the degradation process is predominantly confined to the surface. Thus, selection of specific precursors, namely, alkane diols and cyclic α-hydroxyl acid monomer, may be used to tailor crystallinity and degradation of resulting polyesters to achieve a desired surface erosion profile.

Sutures of the present disclosure may be of monofilament or multifilament braids. Fibers used for forming sutures of the present disclosure may be formed using any technique within the purview of those skilled in the art, such as, for example, extrusion, molding and/or solvent casting. In embodiments, the fibers can be extruded through an extruder unit of a conventional type, such as those disclosed in U.S. Pat. Nos. 6,063,105; 6,203,564; and 6,235,869, the entire contents of each of which are incorporated by reference herein.

In embodiments, the suture of the present disclosure may include a yarn made of more than one fiber, which may contain multiple fibers of the same or different materials. Where the sutures are made of multiple fibers, the suture can be made using any known technique such as, for example, braiding, weaving or knitting. The fibers may also be combined to produce a nonwoven suture. The fibers themselves may be drawn, oriented, crinkled, twisted, commingled or air entangled to form yarns as part of the suture forming process. In one embodiment a multifilament suture of the present disclosure can be produced by braiding.

Barbed sutures and placement methods suitable for use according to the present disclosure include those described in U.S. Patent Nos. 5,931,855, and 6,599,310, and U.S. Patent Application Publication Nos. 2003/0074023, 2003/0074023, 2004/0088003, 2004/0060409, and 2004/0060410, the entire disclosures of each of which are incorporated by reference herein.

Barbs may be formed on the surface of the body of a suture utilizing any method within the purview of one skilled in the art. Such methods include, but are not limited to, cutting, molding, and the like. In some embodiments, barbs may be formed by making with acute angular cuts directly into the suture body, with cut portions pushed outwardly and separated from the body of the suture. The depth of the barbs thus formed in the suture body may depend on the diameter of the suture material and the depth of the cut. In some embodiments, a suitable device for cutting a plurality of axially spaced barbs on the exterior of a suture filament may use a cutting bed, a cutting bed vise, a cutting template, and a blade assembly to perform the cutting. In operation, the cutting device has the ability to produce a plurality of axially spaced barbs in the same or random configuration and at different angles in relation to each other. Other suitable methods of cutting the barbs include the use of a laser or manual methods. The suture could also be formed by injection molding, extrusion, stamping and the like. The suture can be packaged in any number of desired pre-cut lengths and in pre-shaped curves.

In embodiments, needles may be attached to sutures for easier penetration into tissue. In order to facilitate needle attachment to a suture of the present disclosure, conventional tipping agents can be applied to the braid. Two tipped ends of the suture may be desirable for attaching a needle to each end of the suture to provide a so-called double armed suture. The needle attachment can be made by any conventional method such as crimping, swaging, etc., including those described in U.S. Pat. Nos. 5,133,738; 5,226,912; and 5,569,302, the disclosures of which are incorporated by reference herein. Sutures may incorporate various sizes, lengths, diameters and needles the like, curved, straight, or otherwise. Additionally, sutures can be packaged in any number of desired pre-cut lengths and in pre-shaped curves.

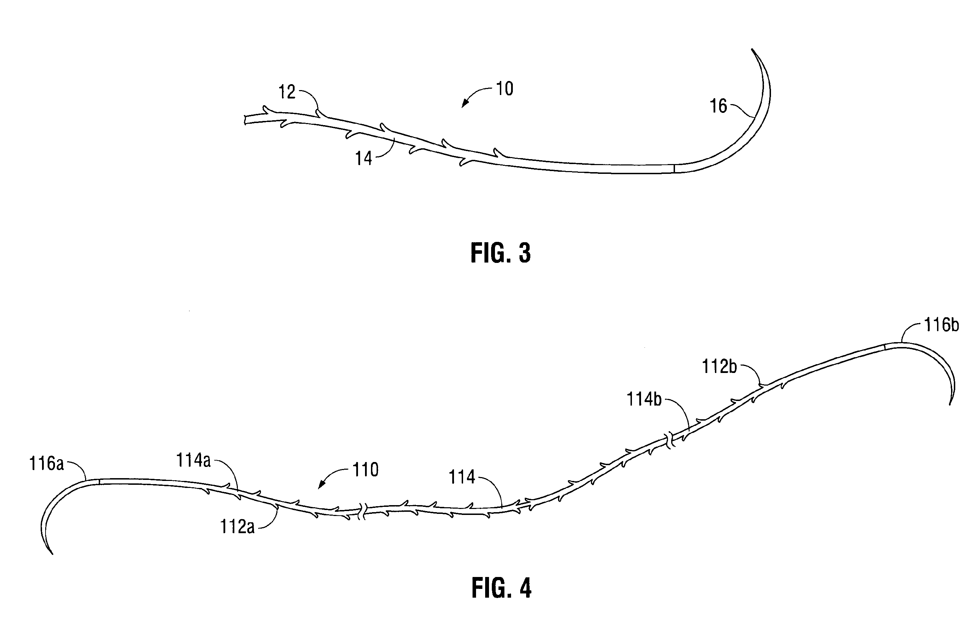

Fig. 3 shows a unidirectional surface eroding suture 10. The suture 10 includes an elongate body 14 and a plurality of surface features 12 extending from the periphery of the body and a needle 16 disposed at a distal end thereof. In embodiments, surface features may be barbs, hooks, latches, protrusions, leaves, teeth and/or combinations thereof. In embodiments, all of the surface features 12 may be aligned to allow the suture 10 to move through tissue in one direction and resist moving through tissue in the opposite direction. The surface features 12 are yieldable toward the body 14 of suture 10. The surface features 12 permit movement of suture 10 through tissue in the direction of movement of the needle 16 but are generally rigid in an opposite direction and prevent and/or restrict movement of suture 10 in the opposite the direction.

Fig. 4 shows a bidirectional surface eroding suture 110. Suture 110 includes an elongate body 114 having a first body portion 114a and a second body portion 114b, a first needle 116a and a second needle 116b for penetrating tissue, and a plurality of surface features 112a and 112b extending from the periphery of the body 114. As discussed above with respect to Fig. 3, the surface features may be barbs, hooks, latches, protrusions, leaves, teeth and/or combinations thereof. The surface features 112a and 112b can be arranged in any suitable pattern (e.g., a helical pattern, parallel rows with a longitudinal axis defined by the suture 110, rings, etc.).

The surface features 112a may be aligned on the first body portion 114a to allow movement of a first end of the suture through tissue in one direction, while surface features 112b may be disposed on the second body portion 114b to allow movement of the second end of the suture in an opposite direction. In particular, surface features 112a on the first body portion 114a permit movement of suture 110 through the tissue in a direction of the first needle 116a and prevent movement of suture 110 in a direction of the second needle 116b. Conversely, surface features 112b on second portion of body 114b permit movement of the suture 110 through the tissue in a direction of the second needle 116b and prevent movement of the suture 110 in a direction of the first needle 116a.

The surface erosion rate is proportional to the surface area of the suture (e.g., suture 10 and suture 110). Thus, the surface erosion rate of the suture may be adjusted by modifying the surface area thereof. In particular, this may be achieved by forming a plurality of surface protrusions, such as barbs and other types listed above. The surface area may also be adjusted by including various grooves and/or depressions on the surface of the suture. The surface features disposed on the surface of the suture increase the surface area, which in turn, increase the surface erosion rate. Thus, increasing the number of surface features, in turn, also increases the erosion rate.

The surface erosion rate may be further fine-tuned by varying the number, configuration, spacing of the surface features. These properties may also be adjusted based on the tissue in which the suture is used, as well as the composition and geometry of the material utilized to form the suture. The erosion rate may also be adjusted by selecting surface features having a specific size, structure, shape, surface area, and protrusion angle (e.g., included angle between the surface features and the suture body). For example, larger or fuller-tipped surface features erode slower than smaller or thinner surface features having similar surface area, allowing the fuller-tipped surface features to remain attached to the tissue. In embodiments, a combination of large and small surface features on the same suture may be beneficial, for example when a suture is used in tissue repair with differing layer structures. Use of the combination of large and small surface features with the same suture wherein barb sizes are customized for each tissue layer may ensure maximum anchoring properties. In embodiments, a unidirectional suture as depicted in Fig. 3 may have both large and small surface features; in other embodiments a bidirectional suture as depicted in Fig. 4 may have both large and small surface features.

In embodiments, both the surface eroding materials utilized to form sutures of the present disclosure and the surface are may affect the surface erosion rate of the suture. Sutures of the present disclosure may thus possess an increased surface erosion rate from about 1 % to about 30 % compared with a suture lacking the surface-erodible material and/or surface feature, in embodiments an increased surface erosion rate of from about 2 % to about 15 % compared with a suture lacking the surface-erodible material and/or surface features, in embodiments an increased surface area of from about 1 % to about 30 % compared with a suture lacking the surface-erodible material and/or surface features.

In embodiments, sutures of the present disclosure may maintain their structural integrity, i.e., 80% of their original strength, after implantation for periods of time ranging approximately from about 3 to about 28 days, in embodiments from about 5 to about 21 days, in other embodiments from about 7 to about 14 days. In some embodiments, the formation of surface features on a surface eroding suture body may be utilized to increase the degradation time of a suture from about 1% to about 25%, in embodiments from about 2% to about 20%, in other embodiments from about 3% to about 10% as compared to a surface eroding suture without surface features.

Sutures fabricated from a surface eroding material in accordance with the present disclosure maintain their structural integrity after implantation (e.g., about 80% of original strength) for a predetermined period of time, depending on the characteristics of the particular material used. Such characteristics include, for example, materials selection, monofilament versus multifilament, to processing conditions (e.g., rate of copolymerization reaction, temperature for reaction, pressure, etc.), and any post-treatment of the resulting sutures, i.e., drying, coating, sterilization, etc.

The sutures of the present disclosure may also include a bioactive agent. A bioactive agent may be impregnated within a polymer resin, combined during the extrusion process or applied to the surface thereof. In embodiments, the bioactive agent may be disposed on the surface of the suture, such as localized in between the barb and the body of the suture, as described in U. S. Provisional Application No. 60/842,763 which is incorporated herein by reference. In other embodiments, a bioactive agent may be otherwise incorporated into the polymer chain or monomer unit (i.e., polymer drug).

Suitable bioactive agents include, for example, biocidal agents, antibiotics, antimicrobial agents, medicants, growth factors, anti-clotting agents, analgesics, anesthetics, anti-inflammatory agents, wound repair agents and the like, and combinations thereof.

Bioactive agents include substances which are beneficial to the patient and tend to promote the wound healing process. For example, a suture can be provided with a bioactive agent which will be released at the sutured site. For example, bioactive agents can be chosen for their antimicrobial properties, capability for promoting wound repair and/or tissue growth, or for specific indications such as thrombosis. In embodiments, combinations of such agents may be applied to or incorporated within a suture of the present disclosure.

The term "antimicrobial agent" as used herein includes an agent which helps the body destroy or resist pathogenic (disease-causing) microorganisms. An antimicrobial agent includes antibiotics, antiseptics, disinfectants and combinations thereof. Antimicrobial agents such as broad spectrum antibiotics (gentamicin sulfate, erythromycin or derivatized glycopeptides) which are slowly released into the tissue can be applied in this manner to aid in combating clinical and sub-clinical infections in a surgical or trauma wound site. In embodiments, suitable antimicrobial agents may be soluble in one or more solvents.

In embodiments, the sutures according to the present disclosure may also be formed from or incorporate polymer drugs (e.g., polydiflunisol, polyaspirin, and protein therapeutics) and biologically modified (e.g., protein, peptide) degradable polymers; and copolymers and combinations thereof.

Classes of antibiotics that can be used as the antimicrobial agent include tetracyclines like minocycline; rifamycins like rifampin; macrolides like erythromycin; penicillins like nafcillin; cephalosporins like cefazolin; beta-lactam antibiotics like imipenem and aztreonam; aminoglycosides like gentamicin and TOBRAMYCIN^{®}; chloramphenicol; sulfonamides like sulfamethoxazole; glycopeptides like vancomycin; quinolones like ciprofloxacin; fusidic acid; trimethoprim; metronidazole; clindamycin; mupirocin; polyenes like amphotericin B; azoles like fluconazole; and beta-lactam inhibitors like sulbactam.

Examples of antiseptics and disinfectants which may be utilized as the antimicrobial agent include hexachlorophene; cationic biguanides like chlorhexidine and cyclohexidine; iodine and iodophores like povidone-iodine; halo-substituted phenolic compounds like PCMX (i.e., p-chloro-m-xylenol) and triclosan (i.e., 2,4,4'-trichloro-2'hydroxy-diphenylether); furan medical preparations like nitrofurantoin and nitrofurazone; methenamine; aldehydes like glutaraldehyde and formaldehyde; and alcohols. In some useful embodiments, at least one of the antimicrobial agents may be an antiseptic such as triclosan.

To promote wound repair and/or tissue growth, one or more bioactive agents known to achieve either or both of these objectives can also be incorporated in the suture as wound repair agents or tissue growth agents. Such materials include any of several human growth factors (HGFs), magainin, tissue or kidney plasminogen activator to cause thrombosis, superoxide dismutase to scavenge tissue-damaging free radicals, tumor necrosis factor for cancer therapy, colony stimulating factor, interferon, interleukin-2 or other lymphokines to enhance the immune system, combinations thereof, and so forth. Still further, surface eroding polymer drugs prepared from condensation polymerization of monomeric or multimeric drugs including, but not limited to non-steriodal anti-inflammatory drugs (NSAIDS) such as salicyclic acid and diflunisal.

Sutures in accordance with this disclosure can also include, for example, biologically acceptable plasticizers, antioxidants and colorants, which can be impregnated into the filament(s) utilized to form a suture of the present disclosure or included in a coating thereon.

As noted above, bioactive agents may be impregnated into the polymer resin or coated on the surface thereof. Bioactive agents may be applied onto a suture of the present disclosure utilizing any method within the purview of one skilled in the art including, for example, dipping, spraying, vapor deposition, brushing, and the like. In embodiments the bioactive agent, such as an antimicrobial agent, may be combined with the resin and extruded.

In other embodiments, a suture, or a portion thereof, may be coated with a biocompatible material which may impart desired handling characteristics. The addition of a coating should not, however, adversely affect the strength and tensile properties of the suture. Suitable coatings which may be utilized are within the purview of one skilled in the art and include, for example, biodegradable coatings such as those disclosed in U.S. Patent Publication No. 20040153125, the entire disclosure of which is incorporated by reference herein.

An "effective antimicrobial amount" of a given component is an amount at which the component hinders the growth of bacteria to diminish or avoid contamination of the wound site.

In embodiments, the antimicrobial degradable coating composition for biocompatible surgical implantable devices is inexpensive, biocompatible, and not subject to excessive diffusion. "Biocompatible" meaning, the ability of a material to perform within an appropriate host response in a specific application (DF Williams 1987), Appropriate host responses include resistance to blood clotting, resistance to bacterial colonization and normal uncomplicated healing.. It is contemplated that biocompatible objects may be associated with some clinically acceptable amounts of toxicity including irritation and/or other adverse reactions in certain individuals.

Such a coating may provide sutures with the combined desirable properties of improved handling characteristics, antimicrobial activity and strength loss which is proportional to mass loss.

In addition to the antimicrobial agents described above, in some embodiments the coating may include fatty acid metal salts which may impart antimicrobial characteristics to the suture.

Where a bioactive agent is included as part of a coating, the bioactive agent and coating components may be added to separate solvents, and the resulting solvent mixtures may then be combined to form a coating solution. In other embodiments, the bioactive agent and coating components may be combined together and then mixed with solvent to form a coating solution or any combination.

The coating can be applied to a suture by any suitable process, e.g., passing the suture through a solution of the coating mixture, past a brush or other coating solution applicator, or past one or more spray nozzles dispensing the suture coating solution. In embodiments, a mixture of methylene chloride, hexane and ethanol may be used as a solvent. The suture wetted with the coating solution may be optionally passed through or held in a drying oven for a time and at a temperature sufficient to vaporize and drive off the solvent. If desired, the suture coating composition can optionally contain additional bioactive agents or components described above, e.g., dyes, antibiotics, antiseptics, growth factors, anti-inflammatory agents, etc.

In embodiments, sutures of the present disclosure may be dyed in order to increase the visibility of the suture in the surgical field. Any dye suitable for incorporation in sutures can be used. Such dyes include, but are not limited to, carbon black, bone black, D&C Green No. 6, and D&C Violet No. 2.

The degradation rate of the resulting sutures may be affected by several factors including the character of the coating composition and the quantity applied. In some embodiments, it may be desirable to further treat the sutures of the present disclosure to obtain the desired rate of degradation. For example, in some embodiments it may be desirable to heat the sutures of the present disclosure to obtain the desired rate of degradation. The heating of the suture may also remove monomers remaining in the polymers utilized to form the sutures. Suitable temperature for heating the sutures can be from about 100° C to about 160° C, in embodiments from about 120° C to about 143° C, for a period of time from about 2 hours to about 24 hours, in embodiments from about 8 hours to about 16 hours. In some embodiments, the heating may take place in a vacuum.

In other embodiments, the rate of degradation of the sutures of the present disclosure may be controlled by exposing them to a plasma treatment, including a low-temperature gas plasma at a pressure substantially below atmospheric for a sufficient period of time. Such methods are within the purview of those skilled in the art and include, for example, the treatment disclosed in U.S. Patent No. 5,236,563, the entire disclosure of which is incorporated by reference herein. In embodiments, the surface treatment may be limited in time to treat the surface layer to a depth of from about 100 to about 1500 Angstroms.

In some cases a tubular insertion device (not shown) may be utilized to introduce a suture in accordance with the present disclosure into tissue. Such a tubular insertion device may have a tubular body in which the suture of the present disclosure is disposed, as well as a distal end and a proximal end. In use, the pointed end of a suture of the present disclosure may be pushed with the distal end of the tubular insertion device through skin, tissue, and the like at an insertion point. The pointed end of the suture and the distal end of the tubular insertion device are pushed through the tissue until reaching an endpoint. The proximal end of the tubular insertion device is then gripped and pulled to remove the insertion device, leaving the suture in place.

Methods for repairing tissue with the sutures of the present disclosure are also provided. The sutures of the present disclosure may be utilized in any cosmetic endoscopic or laparoscopic methods. In addition, sutures of the present disclosure may be utilized to attach one tissue to another including, but not limited to, wound closure. As used herein, the term "wound" means a surgical incision, cut, laceration, severed tissue or accidental wound in human skin or other bodily tissue, or other condition where suturing, stapling, or the use of another tissue connecting device might be required. As used herein, the term "tissue" includes tissues such as skin, bone, muscle, organs, and other soft tissue such as tendons, ligaments and muscle.

In embodiments, sutures of the present disclosure may be held in place without the need for knots. In such cases, tissue located over a suture of the present disclosure placed in vivo may be physically manipulated or massaged into a desired position to enhance the holding of tissue in the desired position. In embodiments, the physical manipulation of tissue located over a suture of the present disclosure may enhance the release of any medicinal agent located on the suture, including any medicinal agent found in the angle between a barb and the body of a suture of the present disclosure. In other embodiments, the "S" or "J" stitching through tissue will activate the surface features, catching tissue and creating mechanical forces preventing the suture from moving backwards and holding without a knot. The term "activate" means causing the surface features to project outwards from suture.

For example, when the suture is used in tissue to repair a wound, the suture is passed through tissue at each of the sides of the wound. The point at one end of the suture, or in some embodiments, the needle, is inserted into a first side of a wound such that the point or needle pierces the tissue and the surface features on the end portion of the suture corresponding to the one end yield toward the body to facilitate movement of the suture through the tissue in the direction of insertion. The other end of the suture may also be inserted into a side of the wound and advanced through the tissue in like manner. The sides or faces of the wound are then moved together along the suture portions within the tissue to close the wound. The surface features of the suture grasp the surrounding tissue on each side of the wound and maintain the edges of the wound in position during healing. The leading ends of the suture protruding from the tissue are then cut and discarded. In one embodiment, ends of the suture in the tissue are made to lie below the surface of the skin by first depressing the skin immediately around the ends and severing the suture body closely against the skin. The skin will rise to cover the ends of the suture.

While the above description contains many specifics, these specifics should not be construed as limitations on the scope of the disclosure, but merely as exemplifications of embodiments thereof. Those skilled in the art will envision many other possibilities within the scope and spirit of the disclosure as defined by the claims appended hereto.
The invention may be described by reference to the following numbered paragraphs:-
1. A process comprising:
   contacting at least one hydrophobic core with at least one α-hydroxy acid ester to form at least one macromer;
   contacting the at least one macromer with at least one diacid spacer to form a surface eroding material; and
   forming a suture from the surface eroding material, the suture including an elongate body with a proximal end and a distal end.
2. The process according to paragraph 1, further comprising adjusting a surface erosion rate of the suture by creating a plurality of surface features.
3. The process according to paragraph 2, wherein the plurality of surface features modifies the surface area of the elongate body.
4. The process according to paragraph 2, wherein the plurality of surface features are selected from the group consisting of barbs, hooks, latches, protrusions, leaves, teeth and combinations thereof.
5. The process according to paragraph 4, wherein the plurality of surface features project from the elongate body towards at least one of the proximal end and the distal end.
6. The process according to paragraph 5, further comprising forming the plurality of surface features having an included angle of less than about 90 degrees between the surface features and the elongate body.
7. The process according to paragraph 1, wherein the at least one hydrophobic core is an alkane diol selected from the group consisting of 1,2-ethanediol, 1,3-propanediol, 1,4-butanediol, 1,5-pentanediol, 1,6-hexanediol, 1,7-heptanediol, 1,8-octanediol, 1,9-nonanediol, 1,10-decanediol, 1,12-dodecanediol, and combinations thereof.
8. The process according to paragraph 1, wherein the at least one α-hydroxy acid ester is selected from group consisting of lactic acid, L-lactide, D-lactide, meso-lactide, glycolic acid, glycolide, epsilon-caprolactone, p-dioxanone, trimethylene carbonate, delta-valerolactone, beta-butyrolactone, epsilon-decalactone, 2,5-diketomorpholine, pivalolactone, alpha-diethylpropiolactone, ethylene carbonate, ethylene oxalate, 3-methyl-1,4-dioxane-2,5-dione, 3,3-diethyl-1,4-dioxan-2,5-dione, gamma-butyrolactone, 1,4-dioxepan-2-one, 1,5-dioxepan-2-one, 1,4-dioxan-2-one, 6,8-dioxabicycloctane-7-one, and combinations thereof.
9. The process according to paragraph 1, further comprising attaching a needle to at least one of the proximal end and the distal end of the elongate body.
10. The process according to paragraph 1, further comprising applying a coating including a bioabsorbable polymer to at least a portion of the suture.
11. The process according to paragraph 1, wherein the suture further includes a bioactive agent or drug.
12. The process according to paragraph 11, wherein the bioactive agent includes biocidal agents, antibiotics, antimicrobial agents, medicants, growth factors, anti-clotting agents, analgesics, anesthetics, anti-inflammatory agents, wound repair agents, polymer drugs and combinations thereof.
13. A process comprising:
   contacting at least one hydrophobic core with at least one α-hydroxy acid ester to form at least one macromer;
   contacting the at least one macromer with at least one diacid spacer to form a surface eroding material;
   forming a suture from the surface eroding material, the suture having an elongate body with a proximal end and a distal end; and
   forming a plurality of surface features on the elongate body.
14. The process according to paragraph 13, wherein the plurality of surface features are selected from the group consisting of barbs, hooks, latches, protrusions, leaves, teeth and combinations thereof.
15. The process according to paragraph 13, wherein the plurality of surface features project from the elongate body towards at least one of the proximal end and the distal end and forming an included angle of less than about 90 degrees between the surface features and the elongate body.
16. The process according to paragraph 13, wherein the at least one hydrophobic core is an alkane diol selected from the group consisting of 1,2-ethanediol, 1,3-propanediol, 1,4-butanediol, 1,5-pentanediol, 1,6-hexanediol, 1,7-heptanediol, 1,8-octanediol, 1,9-nonanediol, 1,10-decanediol, 1,12-dodecanediol, and combinations thereof.
17. The process according to paragraph 13, wherein the at least one α-hydroxy acid ester is selected from group consisting of lactic acid, L-lactide, D-lactide, meso-lactide, glycolic acid, glycolide, epsilon-caprolactone, p-dioxanone, trimethylene carbonate, delta-valerolactone, beta-butyrolactone, epsilon-decalactone, 2,5-diketomorpholine, pivalolactone, alpha-diethylpropiolactone, ethylene carbonate, ethylene oxalate, 3-methyl-1,4-dioxane-2,5-dione, 3,3-diethyl-1,4-dioxan-2,5-dione, gamma-butyrolactone, 1,4-dioxepan-2-one, 1,5-dioxepan-2-one, 1,4-dioxan-2-one, 6,8-dioxabicycloctane-7-one, and combinations thereof.
18. The process according to paragraph 13, further comprising attaching a needle to at least one of the proximal end and the distal end of the elongate body.
19. The process according to paragraph 13, further comprising applying a coating including a bioabsorbable polymer to at least a portion of the suture.
20. The process according to paragraph 13, wherein the suture further includes a bioactive agent or drug.
21. The process according to paragraph 20, wherein the bioactive agent includes biocidal agents, antibiotics, antimicrobial agents, medicants, growth factors, anti-clotting agents, analgesics, anesthetics, anti-inflammatory agents, wound repair agents, polymer drugs and combinations thereof.

## Claims

1. A process comprising:
contacting at least one hydrophobic core with at least one α-hydroxy acid ester to form at least one macromer;
contacting the at least one macromer with at least one diacid spacer to form a surface eroding material; and
forming a suture from the surface eroding material, the suture including an elongate body with a proximal end and a distal end.

2. The process according to claim 1, further comprising adjusting a surface erosion rate of the suture by creating a plurality of surface features.

3. The process according to claim 2, wherein the plurality of surface features modifies the surface area of the elongate body, preferably wherein the plurality of surface features are selected from the group consisting of barbs, hooks, latches, protrusions, leaves, teeth and combinations thereof.

4. The process according to claim 3, wherein the plurality of surface features project from the elongate body towards at least one of the proximal end and the distal end.

5. The process according to any of claims 2 to 5, further comprising forming the plurality of surface features having an included angle of less than about 90 degrees between the surface features and the elongate body.

6. The process according to any preceding claim, wherein the at least one hydrophobic core is an alkane diol selected from the group consisting of 1,2-ethanediol, 1,3-propanediol, 1,4-butanediol, 1,5-pentanediol, 1,6-hexanediol, 1,7-heptanediol, 1,8-octanediol, 1,9-nonanediol, 1,10-decanediol, 1, 12-dodecanediol, and combinations thereof; and/or wherein the at least one α-hydroxy acid ester is selected from group consisting of lactic acid, L-lactide, D-lactide, meso-lactide, glycolic acid, glycolide, epsilon-caprolactone, p-dioxanone, trimethylene carbonate, delta-valerolactone, beta-butyrolactone, epsilon-decalactone, 2,5-diketomorpholine, pivalolactone, alpha-diethylpropiolactone, ethylene carbonate, ethylene oxalate, 3-methyl-1,4-dioxane-2,5-dione, 3,3-diethyl-1,4-dioxan-2,5-dione, gamma-butyrolactone, 1,4-dioxepan-2-one, 1,5-dioxepan-2-one, 1,4-dioxan-2-one, 6,8-dioxabicycloctane-7-one, and combinations thereof.

7. The process according to any preceding claim, further comprising attaching a needle to at least one of the proximal end and the distal end of the elongate body.

8. The process according to any preceding claim, further comprising applying a coating including a bioabsorbable polymer to at least a portion of the suture.

9. The process according to any preceding claim, wherein the suture further includes a bioactive agent or drug, preferably wherein the bioactive agent includes biocidal agents, antibiotics, antimicrobial agents, medicants, growth factors, anti-clotting agents, analgesics, anesthetics, anti-inflammatory agents, wound repair agents, polymer drugs and combinations thereof.

10. A process comprising:
contacting at least one hydrophobic core with at least one α-hydroxy acid ester to form at least one macromer;
contacting the at least one macromer with at least one diacid spacer to form a surface eroding material;
forming a suture from the surface eroding material, the suture having an elongate body with a proximal end and a distal end; and
forming a plurality of surface features on the elongate body.

11. The process according to claim 10, wherein the plurality of surface features are selected from the group consisting of barbs, hooks, latches, protrusions, leaves, teeth and combinations thereof.

12. The process according to claim 10 or claim 11, wherein the plurality of surface features project from the elongate body towards at least one of the proximal end and the distal end and forming an included angle of less than about 90 degrees between the surface features and the elongate body.

13. The process according to any of claims 10 to 12, wherein the at least one hydrophobic core is an alkane diol selected from the group consisting of 1,2-ethanediol, 1,3-propanediol, 1,4-butanediol, 1,5-pentanediol, 1,6-hexanediol, 1,7-heptanediol, 1,8-octanediol, 1,9-nonanediol, 1,10-decanediol, 1,12-dodecanediol, and combinations thereof, and/or wherein the at least one α-hydroxy acid ester is selected from group consisting of lactic acid, L-lactide, D-lactide, meso-lactide, glycolic acid, glycolide, epsilon-caprolactone, p-dioxanone, trimethylene carbonate, delta-valerolactone, beta-butyrolactone, epsilon-decalactone, 2,5-diketomorpholine, pivalolactone, alpha-diethylpropiolactone, ethylene carbonate, ethylene oxalate, 3-methyl-1,4-dioxane-2,5-dione, 3,3-diethyl-1,4-dioxan-2,5-dione, gamma-butyrolactone, 1,4-dioxepan-2-one, 1,5-dioxepan-2-one, 1,4-dioxan-2-one, 6,8-dioxabicycloctane-7-one, and combinations thereof.

14. The process according to any of claims 10 to 13, further comprising attaching a needle to at least one of the proximal end and the distal end of the elongate body, and/or further comprising applying a coating including a bioabsorbable polymer to at least a portion of the suture.

15. The process according to any of claims 10 to 14, wherein the suture further includes a bioactive agent or drug, preferably wherein the bioactive agent includes biocidal agents, antibiotics, antimicrobial agents, medicants, growth factors, anti-clotting agents, analgesics, anesthetics, anti-inflammatory agents, wound repair agents, polymer drugs and combinations thereof.
